(19) Europäisches Patentamt
European Patent Office
Office européen des brevets

(11) Veröffentlichungsnummer: **0 016 446**
**B1**

(12) # EUROPÄISCHE PATENTSCHRIFT

(45) Veröffentlichungstag der Patentschrift: **02.06.82**

(51) Int. Cl.³: **C 07 C 83/02**

(21) Anmeldenummer: **80101369.9**

(22) Anmeldetag: **17.03.80**

(54) **Verfahren zur Herstellung von O,N-disubstituierten Hydroxylaminen.**

(30) Priorität: **22.03.79 DE 2911246**

(43) Veröffentlichungstag der Anmeldung: **01.10.80 Patentblatt 80/20**

(45) Bekanntmachung des Hinweises auf die Patenterteilung: **02.06.82 Patentblatt 82/22**

(84) Benannte Vertragsstaaten: **BE CH DE FR GB NL**

(56) Entgegenhaltungen:
**CHEMISCHE BERICHTE, Band 88, Nr. 1, 1955, Seiten 38–41 Weinheim, DE. W. RIED et al.: »Über heterocyclisch substituierte Aminosäuren. II. Mitteil. Notiz über delta-heterocyclisch substituierte alfa-Oxy- und alfa-Hydroxylamino-valeriansäureester« Seite 39**

(73) Patentinhaber: **BASF Aktiengesellschaft, Carl-Bosch-Strasse 38, D-6700 Ludwigshafen (DE)**

(72) Erfinder: **Linhart, Friedrich, Dr., Leisberg 61, D-6900 Heidelberg (DE)**
Erfinder: **Girgensohn, Bjoern, Dr., Neckarpromenade 38, D-6800 Mannheim 1 (DE)**
Erfinder: **Reissenweber, Gernot, Dr., Pfarrer-Friedrich-Strasse 41, D-6700 Ludwigshafen 29 (DE)**
Erfinder: **Hickmann, Eckhard, Dr., Comeniusstrasse 45, D-6700 Ludwigshafen (DE)**

**EP 0 016 446 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von O,N-disubstituierten Hydroxylaminen

Die Erfindung betrifft ein Verfahren zur Herstellung von O,N-disubstituierten Hydroxylaminen durch Umsetzung von O-substituierten Ketoximen mit Wasserstoff in Gegenwart eines Platinkatalysators und von Mineralsäure unter Druck.

O-Methyl-N-cyclohexylhydroxylamin kann man in Form seines Salzes in mehreren Schritten aus Chlorameisensäureester und Cyclohexylhydroxylamin über die N-Hydroxy- und N-Methoxyverbindung des N-Cyclohexylcarbaminsäureäthylesters herstellen (J. Org. Chem., Band 41 (1976), Seiten 1135 bis 1140). Diese Arbeitsweise ist aufwendig, langwierig, erfordert hohe Mengen an Lösungsmitteln und Hilfsstoffen, zahlreiche Reinigungs- und Aufarbeitungsoperationen in den einzelnen Schritten und ergibt bei schlechten Ausbeuten eine große Menge von Nebenprodukten, die die Umwelt belasten.

Es ist bekannt, daß man am Sauerstoffatom unsubstituierte Hydroxylamine durch drucklose Hydrierung von entsprechenden Ketoximen an Platin-Katalysatoren in Gegenwart von Salzsäure in wäßrig-alkoholischem Medium erhalten kann (Bull. Soc. Chim. France, Band 43 (1928), Seiten 231 bis 237). Diese Methode eignet sich jedoch nur zur Herstellung von O-unsubstituierten Hydroxylaminen. Weiterhin ist bekannt, daß sich O-Alkylketoxime in ähnlicher Weise bei 1 bis 3 bar Wasserstoffdruck zu O,N-Dialkylhydroxylaminen reduzieren lassen (J. Amer. Chem. Soc., Band 52 (1930), Seiten 669 bis 679), wobei nach sehr langen Reaktionszeiten bis zu 44 Stunden nur unbefriedigende Ausbeuten erhalten werden. Die Herstellung von O-Alkyl-N-cycloalkylhydroxylaminen wird nach diesem Verfahren nicht durchgeführt.

Es wurde nun gefunden, daß man O,N-disubstituierte Hydroxylamine der Formel

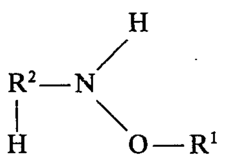

(I)

worin $R^1$ einen aliphatischen Rest und $R^2$ einen gegebenenfalls durch aliphatische Reste substituierten cycloaliphatischen Rest bedeuten, vorteilhaft erhält, wenn man O-substituierte Ketoxime der Formel

$$R^2{=}N{-}O{-}R^1 \qquad \text{(II)}$$

worin $R^1$ und $R^2$ die vorgenannte Bedeutung besitzen mit Wasserstoff im Überschuß und bei einem Druck von 35 bis 500 bar umsetzt.

Die Umsetzung kann für den Fall der Verwendung von O-Methylcyclohexanonoxim durch die folgenden Formeln wiedergegeben werden:

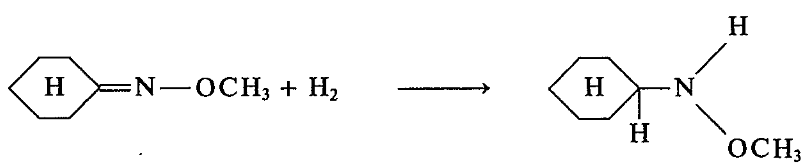

Im Vergleich zu dem bekannten Verfahren liefert das Verfahren nach der Erfindung auf einfacherem und wirtschaftlicherem Wege O,N-disubstituierte Hydroxylamine in besserer Ausbeute und Reinheit. Die Geschwindigkeit der Reaktion ist höher, die Lebensdauer des Katalysators, insbesondere bei den höheren Drücken des erfindungsgemäßen Druckbereiches, vergleichsweise länger. Alle diese vorteilhaften Ergebnisse sind im Hinblick auf den Stand der Technik überraschend. Denn es ist aus Houben-Weyl, Methoden der Organischen Chemie, Georg Thieme Verlag, Stuttgart 1957, Band 11/1, Seite 495, bekannt, daß Oxime sehr leicht über die Hydroxylaminstufe hinweg zu Aminen reduziert werden. Aus diesem Grunde werden bei den bekannten Verfahren in der Regel nur stöchiometrische Mengen Wasserstoff, bezogen auf eingesetztes Oxim, verwendet. Bei dem erfindungsgemäßen Verfahren, bei dem überschüssiger Wasserstoff und hoher Druck verwendet werden, war daher zu erwarten, daß man Amine als Endstoffe erhalten würde. Auch war zu vermuten, daß eine Katalysatorvergiftung die die Lebensdauer des Katalysators verringert, bei höherem Wasserstoffdruck und stärkerer Inanspruchnahme des Katalysators wesentlich schneller eintreten sollte.

Bevorzugte Ausgangsstoffe II und dementsprechend bevorzugte Endstoffe I sind solche, in deren Formeln $R^1$ einen Alkylrest mit 1 bis 7 Kohlenstoffatomen und $R^2$ einen mit 3 oder 2 Alkylgruppen oder insbesondere mit einer Alkylgruppe mit jeweils 1 bis 7 Kohlenstoffatomen substituierten oder insbesondere einen unsubstituierten Cycloalkylenrest mit 5 bis 8 Kohlenstoffatomen bedeuten. Die vorgenannten Reste können noch durch unter den Reaktionsbedingungen inerte Gruppen, z. B.

Alkylgruppen mit 1 bis 4 Kohlenstoffatomen, substituiert sein.

Als Ausgangsstoffe II kommen z. B. in Betracht: O-Methyl-, O-Äthyl-, O-Propyl-, O-Isopropyl-, O-Butyl-, O-sek.-Butyl-, O-Isobutyl-, O-tert.-Butyl-, O-Pentyl-, O-Hexyl-, O-Heptyl-, O-Octyl-, O-Nonyl-, O-Decyl-cyclohexanonoxim; entsprechende am Sauerstoffatom substituierte Cyclopentanonoxime, Cycloheptanonoxime, Cyclooctanonoxime. Entsprechend am Sauerstoffatom substituierte Cyclohexanonoxime, die außerdem am Cycloalkylring in 2-, 3- oder 4-Stellung durch die Methyl-, Äthyl-, Propyl-, Isopropyl-, Butyl-, sek.-Butyl-, tert.-Butyl-, iso-Butyl-gruppe einfach oder gleich oder unterschiedlich in 2,3-Stellung, 2,4-Stellung, 2,5-Stellung, 2,6-Stellung, 3,4-Stellung, 3,5-Stellung zweifach oder gleich oder unterschiedlich in 2,3,4-, 2,3,5-, 2,3,6-, 3,4,5-, 2,4,6- oder 3,4,6-Stellung dreifach substituiert sind; entsprechende am Sauerstoffatom und am Cycloalkylring substituierte Cyclopentanonoxime, Cycloheptanonoxime, Cyclooctanonoxime.

Die Umsetzung wird im allgemeinen bei einer Temperatur von $-70$ bis $+100°$ C, vorzugsweise von $-50$ bis $+50°$ C, insbesondere bei $-20$ bis $+30°$ C, unter einem Druck von 35 bis 500, vorzugsweise von 40 bis 300, insbesondere 40 bis 150 bar, kontinuierlich oder diskontinuierlich durchgeführt. Bei Anwendung eines Druckes von nur 40 bar verläuft die Reaktion bereits mit sehr guter Ausbeute, aber der Katalysator hat eine geringere Lebensdauer als bei Anwendung eines Wasserstoffdruckes von 120 bar. Der Druck kann durch den Eigendruck des Reaktionsgemisches, insbesondere des überschüssigen Wasserstoffs, und gegebenenfalls durch Einleiten inerter Gase, z. B. Stickstoff, eingestellt werden. Zweckmäßig verwendet man unter den Reaktionsbedingungen inerte Lösungsmittel. Als Lösungsmittel kommen z. B. in Frage: Alkanole und Cycloalkanole wie Äthanol, Methanol, n-Butanol, Isobutanol, tert.-Butanol, Glykol, n-Propanol, Isopropanol, Amylalkohol, Cyclohexanol, 2-Methyl-4-pentanol, Äthylenglykolmonoäthyläther, 2-Äthylhexanol, Methylglykol, vorteilhaft Alkanole mit 1 bis 4 Kohlenstoffatomen, insbesondere Methanol, Äthanol; und entsprechende Gemische miteinander und/oder mit Wasser. Zweckmäßig verwendet man das Lösungsmittel in einer Menge von 100 bis 10 000 Gewichtsprozent, vorzugsweise von 400 bis 2000 Gewichtsprozent, bezogen auf Ausgangsstoff II.

Die Umsetzung wird in Gegenwart von Säure, vorteilhaft mit einer Menge von 1 bis 10, insbesondere von 1 bis 5 Äquivalenten Säure, bezogen auf 1 Mol Ausgangsstoff II, durchgeführt. Beispielsweise sind folgende Säuren geeignet: Chlorwasserstoff, Bromwasserstoff, Jodwasserstoff, Schwefelsäure, Phosphorsäure. Bei verdünnten, wäßrigen Säuren sind 5- bis 98gewichtsprozentige Säuren, z. B. 20- bis 38gewichtsprozentige Salzsäure oder 20- bis 98gewichtsprozentige Schwefelsäure, vorteilhaft. Bevorzugt sind Salzsäure und Schwefelsäure.

Man führt die Umsetzung in Gegenwart von Platin und/oder seinen Verbindungen, im allgemeinen in einer Menge von 0,1 bis 10 Gewichtsprozent, vorzugsweise 0,5 bis 5 Gewichtsprozent Platin als Metall, und/oder berechnet als Platin in Gestalt seiner, insbesondere feinverteilten, Verbindungen, bezogen auf Ausgangsstoff II, durch. Es kommen feinverteiltes Metall oder geformtes Metall, z. B. als Bänder, Netze, in Betracht. Ebenfalls können z. B. als Katalysatoren Platinmohr, Platinschwamm, Platinpulver, Platinoxid, Platinbromid, -arsenid, -chlorid, -nitrat, -udid, -oxid, -sulfid, -sulfat oder Komplexsalze wie Tetrachlorplatinate, Tetraamin- oder Diaminplatinchloride, Hexachlorplatin, verwendet werden. Auch Legierungen von Platin mit anderen Metallen, z. B. Rhodium, zweckmäßig von einem Anteil von 0 bis 15 Gewichtsprozent, bezogen auf die Legierung, kommen in Betracht. Vorteilhaft können die genannten Katalysatoren auch in bekannter Weise auf Träger, z. B. Aktivkohle, Bariumsulfat, Graphit, Bimsstein, Asbest, Kieselgel, Tonerde, Aluminogele oder Zeolithe aufgebracht und solche Trägerkatalysatoren für die Hydrierung verwendet werden. Als Platinoxid wird insbesondere das nach der Verfahrensweise von Adams hergestellte $PtO_2 \cdot H_2O$ (J. Am. Chem. Soc. 45, 2175 bis 2178 (1923)) verwendet. Die Herstellung solcher Trägerkatalysatoren kann in beliebiger Weise, z. B. durch Tränken des Trägers mit entsprechenden Lösungen der Platinsalze, durch Verkneten bzw. Mischen unter Vermahlen der Komponenten, erfolgen. Bezüglich Details der Herstellung von Katalysatoren, insbesondere Trägerkatalysatoren, wird auf Houben-Weyl, Methoden der Organischen Chemie, Band 4/2, Seiten 137 ff, verwiesen. Die Umsetzung wird mit einer über die Stöchiometrie hinausgehenden Menge an Wasserstoff, zweckmäßig in einer Menge von 5 bis 10 000, vorzugsweise von 50 bis 1000 Mol, je Mol Ausgangsstoff II, durchgeführt. Man kann den Wasserstoff kontinuierlich oder diskontinuierlich der Reaktion zuführen und/oder den Katalysator selbst nach einer bestimmten Reaktionszeit wieder frisch mit Wasserstoff beladen.

Die Reaktion kann wie folgt durchgeführt werden: Ein Gemisch von Ausgangsstoff II, Säure, Wasserstoff, Hydrierkatalysator und Lösungsmittel wird während 1 bis 6 Stunden bei der Reaktionstemperatur und dem Reaktionsdruck gehalten. Dann wird aus dem Reaktionsgemisch der Endstoff in üblicher Weise, z. B. durch Filtration, Eindampfen des Filtrats, Lösen des Rückstands in Wasser, Extraktion des wäßrigen Gemisches, abgetrennt.

Die nach dem Verfahren der Erfindung herstellbaren O,N-disubstituierten Hydroxylamine I sind wertvolle Ausgangsstoffe für die Herstellung von Farbstoffen, Pflanzenschutzmitteln und Pharmazeutika. So kann man durch Umsetzung der O,N-disubstituierten Hydroxylamine mit Diketen und anschließende Reaktion mit $\alpha$-Hydroxyaldehyden oder $\alpha$-Hydroxyketonen die in der US-Patentschrift 3 993 772 beschriebenen N-Methoxy-N-cycloalkyl-2-methyl-3-furancarboxamide und die in der deutschen Patentschrift 2 455 082 beschriebene O-Methyl-N-cyclohexyl-2,5-dimethylfuran-3-hydroxamsäure herstellen. Diese Stoffe sind, wie in den beiden Patentschriften erläutert wird, aufgrund ihrer Wirkung gegen pflanzenpathogene Pilze wertvolle Fungizide und finden Anwendung als Pflanzenschutzmittel, Saatgutbeizmittel und Holzschutzmittel.

Die in den folgenden Beispielen aufgeführten Teile bedeuten Gewichtsteile.

### Beispiel 1

O-Methyl-N-cyclohexylhydroxylamin

a) Umsetzung: In einem Druckgefäß werden 124,5 Teile O-Methylcyclohexanonoxim, 800 Teile Äthanol, 116 Teile 85prozentige Schwefelsäure und 1 Teil Katalysator Platin/Aktivkohle (10 Gew.-% Pt, bezogen auf Kohle) unter guter Durchmischung mit Wasserstoff bei 20° C unter einem Druck von 40 bar behandelt. 10 Teile Wasserstoff werden insgesamt verwendet. Nach 4 Stunden wird kein Wasserstoff mehr aufgenommen. 2,1 Teile Wasserstoff werden verbraucht. Nach dem Entspannen wird der Katalysator abfiltriert, das Filtrat eingedampft, der Rückstand in Wasser gelöst, mit Cyclohexan extrahiert, der Extrakt mit Ammoniak alkalisch gestellt und das ausgefallene Öl mit Methylenchlorid aufgenommen, getrocknet, eingeengt und destilliert. Man erhält 110 Teile O-Methyl-cyclohexylhydroxylamin (87% der Theorie) vom Kp 65° C (25 mbar).

b) Vergleich: In einem Rührgefäß werden 127 Teile O-Methyl-cyclohexanonoxim, 800 Teile Äthanol, 100 Teile 37gewichtsprozentige Salzsäure und 1,3 Teile eines Platin-Aktivkohle-Katalysators (10 Gew.-% Platin, bezogen auf Kohle) vorgelegt. Unter gutem Rühren wird Wasserstoff unter einem Druck von 1,5 bar zugegeben. 5 Teile Wasserstoff werden insgesamt verwendet. Nach 22 Stunden bei 20°C und 1,5 bar sind 2 Teile Wasserstoff verbraucht. Das Filtrat wird eingedampft, der Rückstand in Wasser gelöst und durch Zugabe von Ammoniak alkalisch gestellt. Es wird kein O-Methyl-N-cyclohexylhydroxylamin erhalten.

### Beispiel 2

In einem Druckgefäß werden 500 Teile Äthanol, 147 Teile 98gewichtsprozentige Schwefelsäure, 27 Teile Wasser, 155 Teile O-Isopropylcyclohexanonoxim und 1 Teil Katalysator Platin/Aktivkohle (10 Gew.-% Pt, bezogen auf Kohle) unter guter Durchmischung mit Wasserstoff bei 20°C unter einem Druck von 40 bar während 3 Stunden behandelt. 20 Teile Wasserstoff werden insgesamt verwendet. 2,1 Teile Wasserstoff werden verbraucht. Dann filtriert man den Katalysator ab und dampft das Filtrat ein. Der Rückstand wird in Wasser gelöst, mit Cyclohexan extrahiert, der Extrakt mit Soda alkalisch gestellt und das sich abscheidende Öl nach dem Trocknen destilliert. Man erhält 139 Teile O-Isopropyl-N-cyclohexylhydroxylamin (89% der Theorie) vom Siedepunkt 86° C (20 mbar).

### Beispiel 3

In einem Druckgefäß werden 258 Teile O-Methylcyclohexanonoxim, 800 Teile Äthanol, 196 Teile 98gewichtsprozentige Schwefelsäure und 2,5 Teile Katalysator Platin/Aktivkohle (5 Gew.-% Pt, bezogen auf Kohle) vorgelegt und unter kräftigem Rühren bei 20°C und einem Wasserstoffdruck von 120 bar bis zur Druckkonstanz hydriert. 50 Teile Wasserstoff werden insgesamt verwendet. 4,1 Teile Wasserstoff werden verbraucht. Nach Aufarbeitung analog Beispiel 1a) erhält man 230 Teile O-Methyl-N-cyclohexylhydroxylamin (89% der Theorie).

Unter Verwendung des gleichen Katalysators werden bei sonst gleichen Bedingungen für zehn nachfolgende Hydrierungen folgende Ausbeuten erhalten: 84%; 82%; 85,5%; 84%; 86%; 85%; 82%; 84%; 86%; 85%.

### Beispiel 4

In einem Druckgefäß vereinigt man 127 Teile O-Methylcyclohexanonoxim, 500 Teile Äthanol, 100 Teile 37gewichtsprozentige, wäßrige Salzsäure und 1,25 Teile Katalysator Platin/Aktivkohle (5 Gew.-% Pt, bezogen auf Kohle). Dann hydriert man das Gemisch 4 Stunden bei 20°C und 70 bar Wasserstoffdruck. 30 Teile Wasserstoff werden insgesamt verwendet. 2,2 Teile Wasserstoff werden verbraucht. Anschließend wird der Katalysator abfiltriert, das Filtrat zum größten Teil eingedampft und in Wasser aufgenommen. Die wäßrige Lösung wird durch Zugabe von Ammoniak alkalisch gestellt und das abgeschiedene Öl wird abgetrennt und destilliert, wobei man 115 Teile O-Methyl-N-cyclohexylhydroxylamin (90% der Theorie) vom Siedepunkt 50° C (14 mbar) erhält.

**Patentanspruch**

Verfahren zur Herstellung von O,N-disubstituierten Hydroxylaminen durch Umsetzung von O-substituierten Ketoximen mit Wasserstoff in Gegenwart eines Platinkatalysators und von Mineralsäure unter Druck, dadurch gekennzeichnet, daß man O,N-disubstituierte Hydroxylamine der Formel

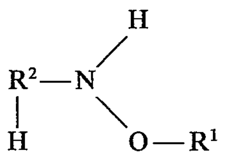

(I)

worin $R^1$ einen aliphatischen Rest und $R^2$ einen gegebenenfalls durch aliphatische Reste substituierten cycloaliphatischen Rest bedeuten, herstellt durch Umsetzung von O-substituierten Ketoximen der Formel

$$R^2{=}N{-}OR^1 \qquad (II)$$

worin $R^1$ und $R^2$ die vorgenannte Bedeutung besitzen, mit Wasserstoff im Überschuß und bei einem Druck von 35 bis 500 bar.

**Claim**

A process for the preparation of an O,N-disubstituted hydroxylamine by reacting an O-substituted ketoxime with hydrogen under pressure in the presence of a platinum catalyst and of a mineral acid, characterized in that an O,N-disubstituted hydroxylamine of the formula

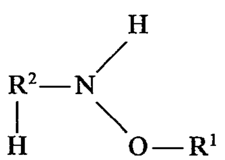

(I)

where $R^1$ is an aliphatic radical and $R^2$ is a cycloaliphatic radical which may or may not be substituted by aliphatic radicals, is prepared by reacting an O-substituted ketoxime of the formula

$$R^2{=}N{-}OR^1 \qquad (II)$$

where $R^1$ and $R^2$ have the above meanings, with excess hydrogen under a pressure of from 35 to 500 bar.

**Revendication**

Procédé de préparation d'hydroxyl-amines O,N-disubstituées par réaction de cétoximes O-substitués sous pression avec l'hydrogène, en présence d'un catalyseur au platine et d'un acide minéral, caractérisé en ce que des hydroxyl-amines O,N-disubstituées de la formule

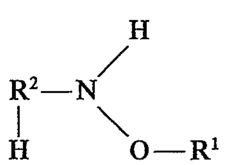

(I)

dans laquelle $R^1$ représente un groupe aliphatique et $R^2$ un groupe cyclo-aliphatique éventuellement substitué par des groupes aliphatiques, sont préparées par la réaction de cétoximes O-substitués de la formule

$$R^2{=}N{-}OR^1 \qquad (II)$$

dans laquelle $R^1$ et $R^2$ possèdent la signification définie, sous une pression de 35 à 500 bars avec de l'hydrogène en excès.